# EUROPEAN PATENT SPECIFICATION

(11) **EP 0 828 753 B1**
(45) Date of publication and mention of the grant of the patent: **26.03.2003**
(21) Application number: 96917365.7
(22) Date of filing: 31.05.1996
(51) Int. Cl.: C07K 1/36, C07K 1/30, C12N 9/00

(54) **Al/Fe-TREATMENT OF A PROTEIN SOLUTION, FOLLOWED BY MEMBRANE CONCENTRATION**
BEHANDLUNG EINER PROTEINLÖSUNG MIT A1/Fe UND ANSCHLIESENDE MEMBRANKONZENTRATION
TRAITEMENT D'UNE SOLUTION PROTEIQUE AVEC UN COMPOSE D'AL/FE, SUIVI D'UNE CONCENTRATION MEMBRANAIRE

(30) Priority: 02.06.1995 DK 62695
(43) Date of publication of application: 18.03.1998
(73) Proprietor: Novozymes A/S, 2880 Bagsvaerd (DK)
(72) Inventor: NIELSEN, Torben Kjaersgaard, Novo Nordisk a/s, DK-2880 Bagsvaerd (DK); LAUSTSEN, Mads Aage, Novo Nordisk a/s, DK-2880 Bagsvaerd (DK); NIELSEN, Niels-Viktor, DK-2880 Bagsvaerd (DK)
(86) International application number: DK9600229
(87) International publication number: WO96038469

(56) References cited:
- EP-A- 0 277 043
- WO-A-90/13632
- WO-A-92/09687
- WO-A-94/01537
- US-A- 3 795 586
- US-A- 4 894 444
- EUR. J. BIOCHEM., Volume 189, 1990, ALEXANDER McPHERSON, "Current Approaches to Macromolecular Crystallization", pages 1-23.

## Description

### TECHNICAL FIELD

The present invention relates to a simple and effective method for purification of a protein, in particular an enzyme, obtained from a protein solution, e.g., a fermentation broth.

### BACKGROUND ART

Use of Al-compounds for precipitation of impurities from fermentation broths prior to evaporation and amorphous salt precipitation is known (for reference see WO 94/01537 and US-A-3,795,586). However, vacuum evaporation is from an energy consumption view expensive. Further, only a small concentration factor is possible in the evaporation process due to a high salt and a low molecular weight component level in a fermented solution. An amorphous salt precipitation with, e.g., Na₂SO₄ or (NH₄)₂SO₄, is highly salt consuming and the process is not an efficient unit operation for protein purification.

Membrane concentration, e.g., UF concentration, is widely used for protein concentration as the process by leaching out salts and small components potentially should give a more efficient concentration than what is possible when using a vacuum evaporation. However, the membrane filtration is often hampered capacity wise, why large seize equipment and thereby high energy consumption is necessary, and further often hampered efficiency wise allowing only for a limited concentration factor. It is therefore a desideratum for the art to develop procedures to overcome the above mentioned problems.

WO 90/13632 describes a process for cleaning enzyme liquid concentrates by adding to the enzyme solution an aluminium salt at a pH between 5 and 11, and, if desired, other precipitating agents.

WO 92/09687 describes a process for separating exocellular proteins from microorganisms from a filtered fermentation liquor. The removal of solids is improved while retaining the useful substance.

### SUMMARY OF THE INVENTION

It has surprisingly been found that a fermentation broth which has been treated with a soluble Fe and/or Al compound is particularly well suited for membrane concentration resulting in a significant capacity gain.

The present invention provides a method for purification of a protein obtained from an aqueous protein solution comprising
(a) treatment of the protein solution with a soluble Fe and/or Al compound at a pH between 4 and 9;
(b) treatment of the protein solution with a salt and an organic polymer;
(c) clarification of the protein solution; and
(d) membrane concentration of said protein solution.

### BRIEF DESCRIPTION OF DRAWINGS

The present invention is further illustrated by reference to the accompanying drawings, in which
Fig. 1 shows an ultrafiltration performance of a protease solution at three different aluminate dosings (■ : 0.8 kg NaAlO₂ per 100 kg fermentation broth; +: 1.6 kg NaAlO₂ per 100 kg fermentation broth; *: 2.4 kg NaAlO₂ per 100 kg fermentation broth), the test performed as described in Example 1.
Fig. 2 shows an ultrafiltration performance of an amylase solution at three different aluminate dosings (■ : 0 kg NaAlO₂ per 100 kg fermentation broth; +: 1 kg NaAlO₂ per 100 kg fermentation broth; *: 4.2 kg NaAlO₂ per 100 kg fermentation broth), the test performed as described in Example 2.
Fig. 3 shows an ultrafiltration performance of a protease solution with/without a previous aluminium sulphate treatment (◆: 0.7 kg 30% Al₂(SO₄)₃x18H₂O per 1.54 kg fermentation broth; □: 0 kg Al₂(SO₄)₃x18H₂O per 1.54 kg fermentation broth); the test performed as described in Example 3.
Fig. 4 shows an ultrafiltration performance of a protease solution with/without a previous iron sulphate treatment □: 0.177 kg 30% Fe₂(SO₄)₃x7H₂O per 1.54 kg fermentation broth; ◆: 0 kg Fe₂(SO₄)₃x7H₂O per 1.54 kg fermentation broth); the test performed as described in Example 4.

### DETAILED DISCLOSURE OF THE INVENTION

The present invention provides a method for purification of a protein obtained from an aqueous protein solution comprising
(a) treatment of the protein solution with a soluble Fe and/or Al compound at a pH between 4 and 9;
(b) treatment of the protein solution with a salt and an organic polymer;
(c) clarification of the protein solution; and
(d) membrane concentration of said protein solution.

The method of the invention can be applied to purification of a protein obtained from an aqueous protein solution, in particular to purification of a protein such as an enzyme from a fermentation broth.

In a preferred embodiment, the method is applied to purification of proteases, lipases, amylases, cellulases. and oxidoreductases.

Proteases: Suitable proteases include those of animal, vegetable or microbial origin. Microbial origin is preferred. Chemically or genetically modified mutants are included. The protease may be a serine protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the Fusarium protease described in WO 89/06270.

Preferred commercially available protease enzymes include those sold under the tradenames Alcalase, Savinase, Primase, Durazym, and Esperase by Novo Nordisk A/S (Denmark), those sold under the tradename Maxatase, Maxacal, Maxapem and Properase by Gist-Brocades, those sold under the tradename Purafect and Purafect OXP by Genencor International, and those sold under the tradename Opticlean and Optimase by Solvay Enzymes.

Lipases: Suitable lipases include those of bacterial or fungal origin. Chemically or genetically modified mutants are included.

Examples of useful lipases include a *Humicola lanuginosa* lipase, e.g., as described in EP 258 068 and EP 305 216, a *Rhizomucor miehei* lipase, e.g., as described in EP 238 023, a *Candida* lipase, such as a *C. antarctica* lipase, e.g., the *C. antarctica* lipase A or B described in EP 214 761, a *Pseudomonas* lipase such as a *P. alcaligenes* and *P. pseudo-alcaligenes* lipase, e.g., as described in EP 218 272, a *P. cepacia* lipase, e.g., as described in EP 331 376, a P. stutzeri lipase, e.g., as disclosed in GB 1,372,034, a P. fluorescens lipase, a *Bacillus* lipase, e.g., a *B. subtilis* lipase (Dartois et al., (1993), Biochemica et Biophysica acta 1131, 253-260), a *B. stearothermophilus* lipase (JP 64/744992) and a *B. pumilus* lipase (WO 91/16422).

Furthermore, a number of cloned lipases may be useful, including the *Penicillium camembertii* lipase described by Yamaguchi et al., (1991), Gene 103, 61-67), the *Geotricum candidum* lipase (Schimada, Y. et al., (1989), J. Biochem., 106, 383-388), and various *Rhizopus* lipases such as a *R. delemar* lipase (Hass, M.J et al., (1991), Gene 109, 117-113), a *R. niveus* lipase (Kugimiya et al., (1992), Biosci. Biotech. Biochem. 56, 716-719) and a *R. oryzae* lipase.

Other types of lipolytic enzymes such as cutinases may also be useful, e.g., a cutinase derived from *Pseudomonas mendocina* as described in WO 88/09367, or a cutinase derived from *Fusarium solani pisi* (e.g. described in WO 90/09446).

Especially suitable lipases are such as Ml Lipase™, Luma fast™ and Lipoma™ (Gist-Brocades), Lipolase™ and Lipolase Ultra™ (Novo Nordisk A/S), and Lipase P "Amano" (Amano Pharmaceutical Co. Ltd.).

Amylases: Suitable amylases (α or β) include those of bacterial or fungal origin. Chemically or genetically modified mutants are included. Amylases include, for example, a-amylases obtained from a special strain of B. licheniformis, described in more detail in GB-A-1,296,839. Commercially available amylases are Duramyl™, Termamyl™, Fungamyl™ and BAN™ (available from Novo Nordisk A/S) and Rapidase™ and Maxamyl P™ (available from Gist-Brocades).

Cellulases: Suitable cellulases include those of bacterial or fungal origin. Chemically or genetically modified mutants are included. Suitable cellulases are disclosed in US 4,435,307, which discloses fungal cellulases produced from Humicola insolens. Especially suitable cellulases are the cellulases having colour care benefits. Examples of such cellulases are cellulases described in EP-A-0 495 257.

Commercially available cellulases are Celluzyme™ produced by a strain of Humicola insolens, (Novo Nordisk A/S), and KAC-500(B)™ (Kao Corporation).

Oxidoreductases: Oxidoreductases are defined and described in, e.g., "Enzyme Nomenclature 1992, Academic press, Inc., San Diego". They belong to a class of enzymes that catalyses transfer of electrons from one substance to another (oxidation-reduction). Oxidoreductases include dehydrogenases, reductases, oxidases, transhydrogenases, catalases, peroxidases, and oxygenases.

More specific examples include horseradish peroxidase, ligninases and other peroxidases, and oxidases such as laccases.

Examples of microorganism genera which may be used for production of suitable oxidoreductases are: *Trametes,* *Rhizoctonia, Pseudomonas, Bacillus, Streptomyces, Hygrophorus, Coprinus, Polyporus, Candida, Curvularia, Cercospora, Mycoliophtora, Aspergillus*, and *Scytalidium.* The invention, however, is not restricted to enzymes derived from the above mentioned taxa. All microorganisms producing oxidoreductases with the desired properties may be used in relation to this invention.

The oxidoreductase is preferably a laccase (EC 1.10.3.2), a catalase (EC 1.11.1.6), a peroxidase (EC 1.11.1.7), or an oxidase.

A suitable peroxidase is one producible by a microorganism such as a fungus or a bacterium. In a preferred embodiment, the peroxidase is derived from *Coprinus*, e.g., *C. cinereus* or *C. macrorhizus*, or from *Bacillus*, e.g., *B. pumilus,* particularly a peroxidase according to International Patent Application WO 91/05858.

The enzyme may furthermore be one which is producible by a method comprising cultivating a host cell transformed with a recombinant DNA vector which carries a DNA sequence encoding said enzyme as well as DNA sequences encoding functions permitting the expression of the DNA sequence encoding the enzyme in a culture medium under conditions permitting the expression of the enzyme and recovering the enzyme from the culture.

Particularly, a recombinantly produced peroxidase is a peroxidase derived from a *Coprinus* sp., in particular *C. macrorhizus* or *C. cinereus* according to WO 92/16634.

Catalases are known both from animal sources (e.g. cow liver) and from many different microorganisms. JP-A-2 076 579 discloses catalase from *Aspergillus niger* strain NFAG-2. GB-A-2,216,149 discloses catalase from *Penicillium.* In a preferred embodiment of the invention the catalase is obtained from strains of *Scytalidium* and *Humicola* as described in WO 92/17571.

The method of the invention may be applied to an untreated fermentation broth or to a fermentation broth that has first been subjected to, e.g., a pH adjustment, a temperature adjustment, a water dilution and/or one or more solid/liquid separatory techniques such as flocculation, centrifugation, filtration or micro filtration.

According to the invention it has been found that an addition of a soluble Fe and/or Al compound to a protein solution results in a liquid which is surprisingly well fitted for membrane concentration as the flux as well as the purity of the protein is increased.

Addition of a soluble Fe and/or Al compound to a fermentation broth will give an insoluble precipitate mainly consisting of Al and/or Fe hydroxide and impurities such as carbohydrates if the pH of the fermentation broth is between 4 and 9. It is important that the pH of the fermentation broth is kept between pH 4 and pH 9. Normally the precipitation process proceeds in the most efficient manner at lower pH-values. However, many proteins such as most enzymes exhibit low stability at low pH-values. Thus, a compromise pH-value with a reasonably efficient process and a reasonably good protein stability has to be chosen.

It is to be understood that the addition of a soluble Al/Fe compound to the fermentation broth with subsequent precipitation of the hydroxide will change the pH, but pH should be readjusted to pH 4 - 9 all through the precipitation phase and a sufficient time after until the pH of the solution is stabilized within the pH range 4-9.

It may, in some cases, be an advantage to use a mixture of acidic and alkaline Al/Fe-compounds in order to minimize the amount of acid/base needed for the pH adjustment, preferably a pH adjustment is not necessary at all. An example of such a combination of an acidic salt and an alkaline salt is sodium aluminate and aluminium sulphate.

If a pH adjustment is necessary any acid or base may be used, but formic acid or acetic acid are preferred as acids.

The minimum amount of an Al/Fe compound which will precipitate no more than a negligible amount of impurities is dependent upon the kind and concentration of the impurities but the minimum amount will typically be 0.02 moles Al and/or Fe per liter of protein solution, preferably 0.04 moles Al and/or Fe per liter of protein solution.

The maximum amount of an Al/Fe compound which will precipitate no more than a negligible amount of protein is dependent upon the kind and concentration of the protein, but the maximum amount will typically be around 1.2 moles Al and/or Fe per liter of protein solution, preferably 1.0 moles Al and/or Fe per liter of protein solution.

According to the invention any soluble Fe or Al compound or any mixture thereof may be used, in particular Al₂(SO₄)₃, NaAlO₂, K₂Al₂O₄, Al(NO₃)₃, AlCl₃, Al-acetate, Al-formate, polymer aluminiumhydroxychloride (e.g., EKOFLOCK available from Boliden), Fe₂(SO₄)₃, Fe(III)-formate, Fe(III)-acetate, Fe(II)-formate and Fe(II)-acetate.

In WO 94/01537 it is disclosed that in a preferred embodiment the mixture comprising soluble aluminate also contains a water miscible solvent such as an alcohol. We have found that an optimal precipitation is facilitated by adding one or more flocculating agents.

According to the invention useful flocculating agents are salts such as Ca- and Mg-salts, in particular phosphates, sulphates or chlorides, e.g., calcium chloride, and inorganic and/or organic polymers which may be cationic, anionic or nonionic, in particular a combination of cationic and anionic polymers.

Before a membrane concentration of the protein solution, e.g., the fermentation broth, can take place the Al/Fe precipitate should be removed by one or more clarification processes which will typically be a centrifugation, a filtration, a microfiltration or a combination thereof. The Al/Fe precipitate may conveniently be removed by drumfiltration followed by a filter press filtration as described in Examples 1 and 2.

According to the invention the membrane concentration may be performed using any membrane equipment known in the art, but it is preferred that the membrane concentration is done using ultrafiltration techniques such as hollow fiber, spiral rounds or plate and frame units. The membranes may be made of a variety of materials such as polysulfone membranes. The preferred cut off value will depend on the properties of the protein in question but usually a cut off value in the interval of from 1 to 100 kD is preferred.

The protein concentrate achieved according to the invention may be further purified in a variety of ways such as by using chromatographic methods, adsorption and/or crystalli-zation processes.

The invention is further illustrated in the following examples which are not intended to be in any way limiting to the scope of the invention as claimed.

### EXAMPLE 1

The following example shows the unique correlation between the use of aluminate and the improvement of capacity (flux) in the following ultrafiltration on polysulfone membrane when purificating a protease from a fermentation broth.

A fermentation broth containing protease (Savinase™) from Bacillus lentus, obtained as described in US-A-3,723,250, was subjected to the method of the invention.

Three tests were performed with three different levels of aluminate in the following way:

After fermentation water and calcium chloride was added to the protease containing broth in the amounts given in Table 1. Then sodium aluminate (dosed as a 20% w/v solution) was added (0.8 kg per 100 kg broth in test A; 1.6 kg per 100 kg broth in test B and 2.4 kg per 100 kg broth in test C) while at the same time pH of the solution was kept at pH 8 by adding formic acid. Lastly the flocculating agents (Superfloc C 521 and Superfloc A 130) were added in the amounts given in Table 1:

**Table 1**

| Chemicals | Test A | Test B | Test C |
|---|---|---|---|
| Broth | 100 kg | 100 kg | 100 kg |
| Water | 219 kg | 218 kg | 213 kg |
| CaCl₂x2H₂O | 2 kg | 2 kg | 2 kg |
| NaAlO₂ (53-55% Al₂O₃) | 0.8 kg | 1.6 kg | 2.4 kg |
| 20% Superfloc C 521 | 2 kg | 2 kg | 2. kg |
| 0.1% Superfloc A 130 | 20 kg | 20 kg | 25 kg |

Hereafter the so treated fermentation broth was drum-filtered and further filtered on a filter press. Finally the solution was ultrafiltered at 7-15°C on a 18 m² DDS 35 L plate and frame system with GR 61 PP membranes. The membranes were made from polysulfone with a cut off value of approx. 20,000 D.

The enzyme concentration of the drum filtrate in test A, B and C was at the same level.

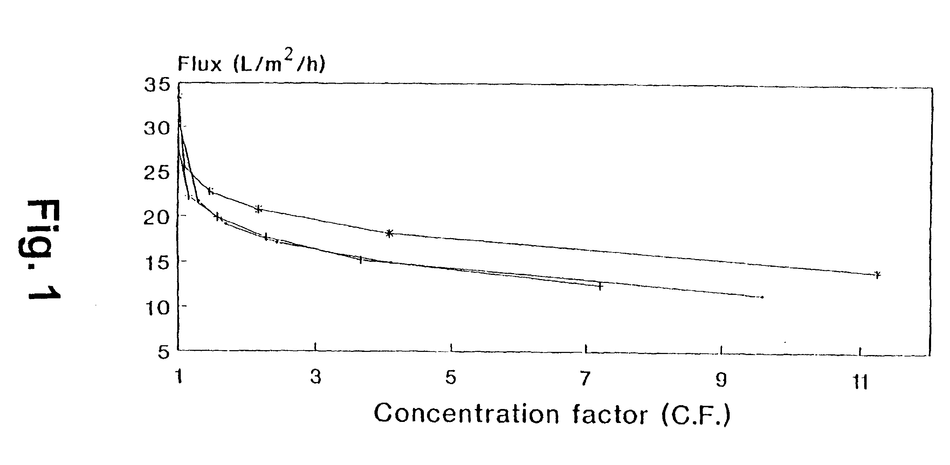

The flux characteristics of the three tests are shown in Figure 1 (^{■}: 0.8 kg NaAlO₂ per 100 kg fermentation broth (test A); +: 1.6 kg NaAlO₂ per 100 kg fermentation broth (test B); *: 2.4 kg NaAlO₂ per 100 kg fermentation broth (test C)). Concentration factor (C.F.) was calculated as Vᵢₙᵢₜᵢₐₗ/V_{concentrate}. Relevant flux data are shown in Table 2.

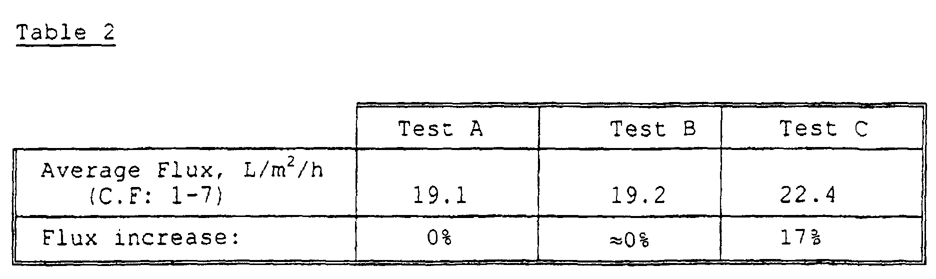

Relevant purity data on the concentrated products (A, B and C) are shown in Table 3.

**Table 3**

| Data | Test A | Test B | Test C |
|---|---|---|---|
| Activity/g dry matter (RI) (non permeable dry matter) | 100% | 114% | 131% |
| OD₄₄₀ₙₘ per activity unit | 100% | 67% | 52% |

The purity data shown in Table 3 clearly indicate that a higher dose of aluminate gives a higher purity of the enzyme per g dry matter and lower concentration of coloured species per activity unit.

### EXAMPLE 2

The following example shows the unique correlation between the use of aluminate and the improvement of capacity (flux) in the following ultrafiteration on hollow fiber module when purifying an amylase from a fermentation broth.

A fermentation broth containing a Bacillus licheniformis amylase (Termamyl™) was subjected to the method of the invention.

Three tests were performed with different levels of aluminate in the following way:

After fermentation water and calcium chloride were added to the amylase containing broth in the amounts given in Table 4. Then sodium aluminate (dosed as a 20% w/v solution) was added (0 kg per 100 kg broth in test A; 1 kg per 100 kg broth in test B and 4.2 kg per 100 kg broth in test C) while at the same time pH of the solution was kept at pH 8 by adding formic acid. Lastly the flocculating agents (Superfloc C 521 and Superfloc A 130) were added in the amounts given in Table 4:

**Table 4**

| Chemicals | Test A | Test B | Test C |
|---|---|---|---|
| Broth | 100 kg | 100 kg | 100 kg |
| Water | 216 kg | 213 kg | 185 kg |
| CaCl₂x2H₂O | 1 kg | 0.5 kg | 1 kg |
| NaAlO₂ (53-55% Al₂O₃) | 0 kg | 1 kg | 4.2 kg |
| 20% Superfloc C 521 | 6.6 kg | 6.6 kg | 3.5 kg |
| 0.1% Superfloc A 130 | 6.5 kg | 10 kg | 40 kg |

The water level was adjusted to give the same enzyme concentration in all of the three flocculated suspensions.

The flocs were separated from the clear liquid by drum filtration without any additional dilution with tab water.

After the drumfiltration the product was further filtered on a filter press to give a NTU value below 100 and finally ultrafiltered at 7-15°C on a Romicon/Koch 2.3 m² hollow fibre module (PM5, 1.1 mm in ID) with a cut off value of 5000 D. The module was operated at a TMP value of approx. 1.2 Bar and a cross flow velocity of approx. 1 m/s. After 10 hours of operation the ultrafiltration was stopped.

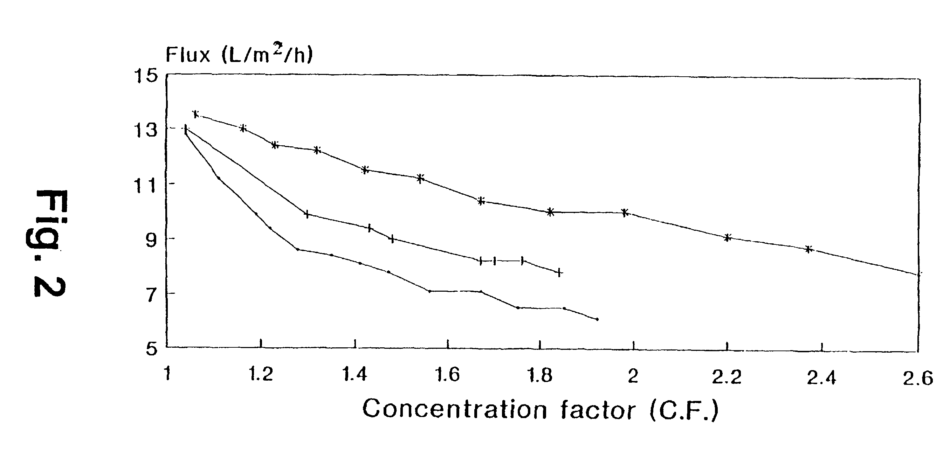

In Figure 2 the flux (L/m²/hour) as a function of the concentration factor is shown for the three different types of filtrates (■ : 0 kg NaAlO₂ per 100 kg fermentation broth (test A); +: 1 kg NaAlO₂ per 100 kg fermentation broth (test B); *: 4.2 kg NaAlO₂ per 100 kg fermentation broth (test C)).

In Table 5, the flux results are summarized.

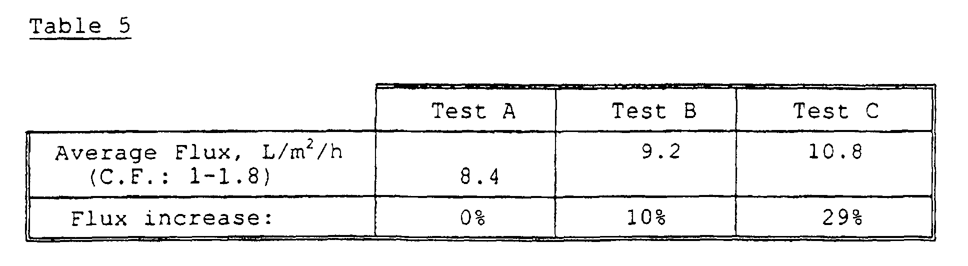

Relevant purity data on the concentrated products are shown in Table 6.

**Table 6**

| Data | Test A | Test B | Test C |
|---|---|---|---|
| Activity/g dry matter (RI) (non permeable dry matter) | 100% | 104% | 116% |
| OD₄₄₀ₙₘ per activity unit | 100% | 73% | 27% |
| g Carbohydrate per activity unit | 100% | 100% | 70% |

The purity data shown in Table 6 clearly indicate that a higher dose of aluminate gives lower carbohydrate levels and a low concentration of coloured species.

### EXAMPLE 3

The following example shows the unique correlation between the use of aluminium sulphate (natural alunogenite) and the improvement of capacity (flux) in the following ultrafiltration on polysulphone membrane when purificating a protease from a fermentation broth.

A fermentation broth containing protease (Savinase™) from Bacillus lentus, obtained as described in US Patent No. 3,723,250 was subjected to the method of the invention.

Two tests were performed. The first test (Test A) without aluminium sulphate and the second test (Test B) with 14% w/w Al₂(SO₄)₃x18H₂O. Both at pH=8.0.

After fermentation water and calcium chloride were added to the procease containing broth in the amounts given in Table 7. Then aluminium sulphate (dosed as a 30% w/w solution) was added (Test B) while at the same time pH of the solution was kept at pH=8 by adding sodium hydroxide. Lastly the flocculating agents (Superfloc C521 and Superfloc A130) were added in the amounts given in Table 7.

**Table 7**

| Chemicals | Test A | Test B |
|---|---|---|
| Broth | 1.54 kg | 1.54 kg |
| Water | 3.0 kg | 1.85 kg |
| CaCl₂x2H₂O | 0.078 kg | 0.078 kg |
| 30 % Al₂(SO₄)₃x18H₂O | 0 kg | 0.70 kg |
| Sodium hydroxide | 0.014 kg | 0.559 kg |
| 20 % Superfloc C521 | 0.028 kg | 0.028 kg |
| 0.1% Superfloc A130 | 0.42 kg | 0.42 kg |

Hereafter the so treated fermentation broths were filtered on a pressure nutsch.

Finally the solutions from Test A and Test 3 were ultrafiltered at 10°C on 27 cm² Amicon cells with PM10 membranes at 3.0 Bar. The membranes were made of polysulphone with a cut off value of approx. 10.000 D.

The enzyme concentration of the filtrates in the two tests were of the same level.

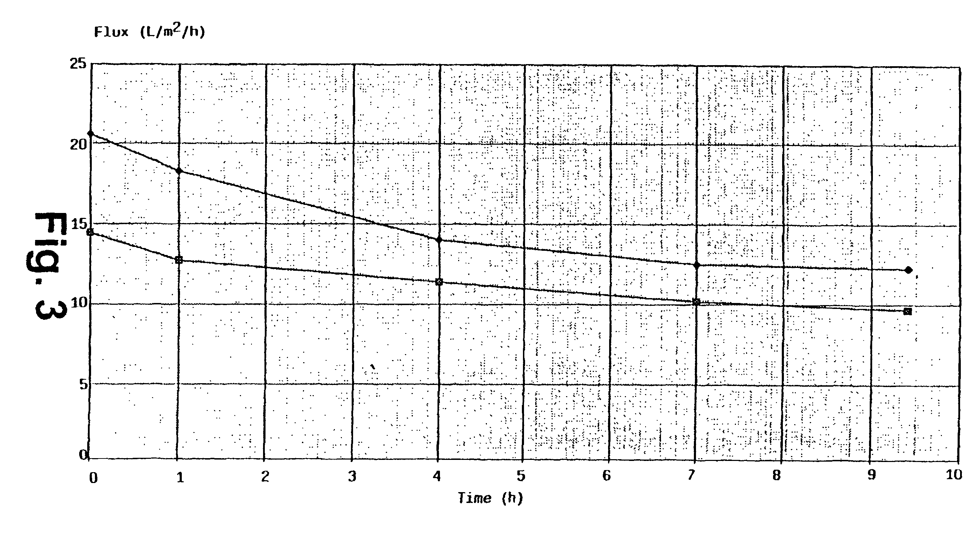

The flux characteristics of the two tests are shown in Figure 3. Concentration factor (C.F.) was calculated as Vᵢₙᵢₜᵢₐₗ/V_{concentrate}, where V is Volume. Yield was calculated as NPU_{final}/NPUᵢₙᵢₜᵢₐₗ*100, where NPU is the amount of Novo Protease Units. Relevant flux data are shown in Table 8.

The protease units were determined as described below:

### Proteolytic Activity

The proteolytic activity may be determined using casein as substrate.

One Casein Protease Unit (NPU) is defined as the amount of enzyme liberating 1 mM of primary amino groups (determined by comparison with a serine standard) per minute under standard conditions, i.e. incubation for 30 minutes at 25°C and pH 9.5.

**Table 8**

| Data | Test A | Test B |
|---|---|---|
| Average flux, L/m²/h (C.F.: 1 - 3) | 11.7 | 15.5 |
| Flux increase (%) | 0 % | 33 % |
| Yield (%) | 100 % | 98 % |

Relevant purity data on the concentrated products (A and B) are shown in Table 9.

**Table 9**

| Data | Test A | Test B |
|---|---|---|
| Activity per g dry matter (RI) | 100 % | 110 % |
| OD₄₄₀ₙₘ per activity unit | 100% | 34% |

The purity data shown in Table 9 clearly indicate that a higher dose of aluminum sulphate gives a higher purity of the enzyme per g dry matter and lower concentration of coloured species per activity unit.

### EXAMPLE 4

The following example shows the unique correlation between the use of iron sulphate and the improvement of capacity (flux) in the following ultrafiltration on polysulphone membrane when purifying a protease from a fermentation broth.

A fermentation broth containing protease (Savinase™) from Bacillus lentus, obtained as described in US Patent No. 3,723,250 was subjected to the method of the invention.

Two tests were performed. The first test (Test A) without iron sulphate and the second test (Test B) with 3.45% w/w Fe₂(SO₄)₃x7H₂O. Both at pH=8.0.

After fermentation water and calcium chloride were added to the protease containing broth in the amounts given in Table 10. Then iron sulphate (dosed as a 30% w/w solution) was added (Test B) while at the same time pH of the solution was kept at pH=8 by adding sodium hydroxide. Last the flocculating agents (Superfloc C521 and Superfloc A130) were added in the amounts given in Table 10.

**Table 10**

| Chemicals | Test A | Test B |
|---|---|---|
| Broth | 1.54 kg | 1.54 kg |
| Water | 3.11 kg | 2.77 kg |
| CaCl₂x2H₂O | 0.078 kg | 0.078 kg |
| 30 % Fe₂(SO₄)₃x7H₂O | 0 kg | 0.177 kg |
| Sodium hydroxide | 0.02 kg | 0.21 kg |
| 20 % Superfloc C521 | 0.028 kg | 0.028 kg |
| 0.1% Superfloc A130 | 0.42 kg | 0.42 kg |

Hereafter the so treated fermentation broth was filtered on a pressure nutsch. Finally the solutions from Test A and Test B were ultrafiltered at 10°C and 3 bar(o) on 27 cm² Amicon cells with PM10 membranes. The membranes were made of polysulphone with a cut off value of appr. 10.000 D.

The enzyme concentration of the filtrates in the two tests were of the same level.

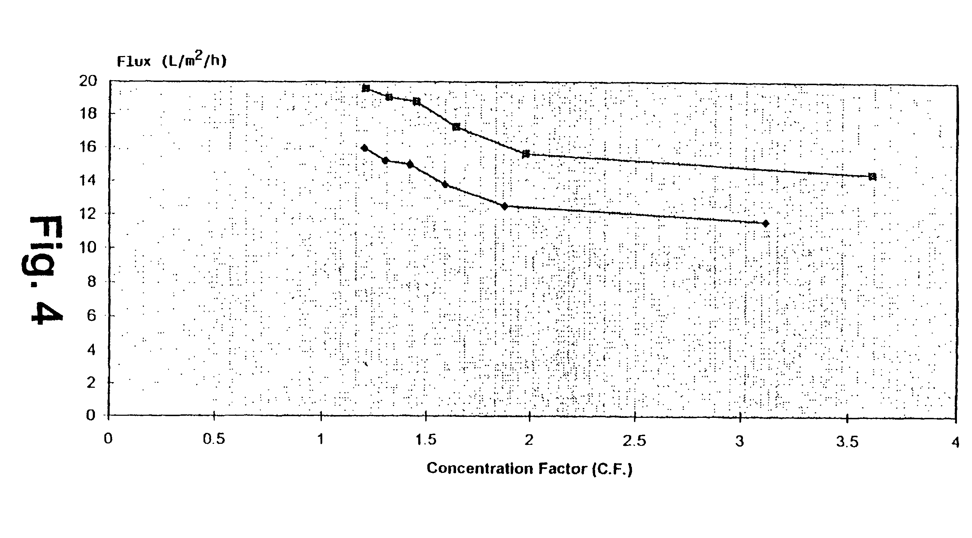

The flux characteristics of the two tests are shown in Figure 4. Concentration factor (C.F.) was calculated as Vᵢₙᵢₜᵢₐₗ/V_{concentrate}, where V is Volume. Yield was calculated as NPU_{final}/NPUᵢₙᵢₜᵢₐₗ*100, where NPU is the amount of Novo Protease Units. Relevant flux data are shown in Table 11.

**Table 11**

| Data | Test A | Test B |
|---|---|---|
| Average flux, L/m²/h (C.F.: 1 - 3) | 13.3 | 16.5 |
| C.F. | 3.11 | 3.61 |
| Flux increase (%) | 0 % | 24 % |
| Yield (%) | 96 % | 97 % |

Relevant purity data on the concentrated products (A and B) are shown in Table 12.

**Table 12**

| Data | Test A | Test B |
|---|---|---|
| Activity per g dry matter (RI) | 100 % | 104 % |
| OD₄₄₀ₙₘ per activity unit | 100 % | 47 % |

The purity data shown in Table 12 clearly indicate that a higher dose of iron sulphate gives a higher purity of the product per g dry matter and lower concentration of coloured species per activity unit.

## Claims

1. A method for obtaining a significant flux increase in a membrane concentration of a protein solution comprising
(a) treatment of the protein solution with a soluble Fe and/or Al compound at a pH between 4 and 9;
(b) treatment of the protein solution with a salt and an organic polymer;
(c) clarification of the protein solution; and
(d) membrane concentration of said protein solution.

2. A method according to claim 1, wherein the protein solution is a fermentation broth.

3. A method according to claim 1, wherein the Al compound is selected from the group consisting of Al₂(SO₄)₃, NaAlO₂, K₂Al₂O₄, AlCl₃, Al(NO₃)₃, Al-acetate, Al-formate and any mixtures hereof.

4. A method according to claim 1, wherein the Fe compound is selected from the group consisting of Fe₂(SO₄)₃, Fe(III)-formate, Fe(III)-acetate, Fe(II)-formate, Fe(II)-acetate and any mixtures hereof.

5. A method according to any preceding claim, wherein the Al and/or Fe compound is added at a concentration of 0.02-1.2 moles per liter of protein solution, preferably at a concentration of 0.04-1.0 moles per liter of protein solution.

6. A method according to claim 1, wherein the clarification is performed by centrifugation, filtration, microfiltration or a combination thereof.

7. A method according to claim 1, wherein the membrane concentration is ultrafiltration.

8. A method according to claim 1, wherein the protein is an enzyme.

9. A method according claim 8, wherein the enzyme is an oxidoreductase, a protease, a lipase, a cellulase or an amylase.

## Patentansprüche

1. Verfahren zum Erhalten einer signifikanten Erhöhung des Flusses in einer Membrankonzentration einer Proteinlösung enthaltend
(a) Behandlung der Proteinlösung mit einer löslichen Fe- und/oder Al-Verbindung bei einem pH zwischen 4 und 9;
(b) Behandlung der Proteinlösung mit einem Salz und einem organischen Polymer;
(c) Klärung der Proteinlösung; und
(d) Membrankonzentration der Proteinlösung.

2. Verfahren nach Anspruch 1, wobei die Proteinlösung eine Fermentationslösung ist.

3. Verfahren nach Anspruch 1, wobei die Al-Verbindung ausgewählt ist aus der Gruppe bestehend aus Al₂(SO₄)₃, NaAlO₂, K₂Al₂O₄, AlCl₃, Al(NO₃)_{3,} Al-Acetat, Al-Formiat und irgendeinem Gemisch hiervon.

4. Verfahren nach Anspruch 1, wobei die Fe-Verbindung ausgewählt ist aus der Gruppe bestehend aus Fe₂(SO₄)₃, Fe(III)-Formiat, Fe(III)-Acetat, Fe(II)-Formiat, Fe(II)-Acetat und irgendeinem Gemisch hiervon.

5. Verfahren nach einem der vorangegangenen Ansprüche, wobei die Alund/oder Fe-Verbindung in einer Konzentration von 0,02-1,2 Molen pro Liter Proteinlösung, vorzugsweise in einer Konzentration von 0,04-1,0 Molen pro Liter Proteinlösung zugefügt wird.

6. Verfahren nach Anspruch 1, wobei die Klärung durch Zentrifugation, Filtration, Mikrofiltration oder eine Kombination hiervon durchgeführt wird.

7. Verfahren nach Anspruch 1, wobei die Membrankonzentration Ultrafiltration ist.

8. Verfahren nach Anspruch 1, wobei das Protein ein Enzym ist.

9. Verfahren nach Anspruch 8, wobei das Enzym eine Oxidoreduktase, eine Protease, eine Lipase, eine Cellulase oder eine Amylase ist.

## Revendications

1. Un procédé pour obtenir une augmentation significative du flux dans une concentration membranaire d'une solution protéique comprenant
a) le traitement de la solution protéique par un composé soluble de Fe et/ou d'Al à un pH compris entre 4 et 9,
b) le traitement de la solution protéique par un sel et un polymère organique,
c) la clarification de la solution protéique et
d) la concentration membranaire de ladite solution protéique.

2. Un procédé selon la revendication 1, dans lequel la solution protéique est un bouillon de fermentation.

3. Un procédé selon la revendication 1, dans lequel le composé d'Al est sélectionné à partir du groupe consistant en Al₂(SO₄)₃, NaAlO₂, K₂Al₂O₄, AlCl₃, Al(NO₃)₃, acétate d'Al, formate d'Al et tout mélange de ces composés.

4. Un procédé selon la revendication 1, dans lequel le composé de Fe est sélectionné à partir du groupe consistant en Fe₂(SO₄)₃, formate de Fe (III), acétate de Fe (III), formate de Fe (II), acétate de Fe (II) et tout mélange de ces composés.

5. Un procédé selon l'une quelconque des revendications précédentes, dans lequel le composé d'Al et/ou de Fe est ajouté à une concentration comprise entre 0,02 et 1,2 mole par litre de solution protéique, de préférence à une concentration comprise entre 0,04 et 1,0 mole par litre de solution protéique.

6. Un procédé selon la revendication 1, dans lequel la clarification est effectuée par centrifugation, par filtration, par micro-filtration ou par une combinaison de ces procédés.

7. Un procédé selon la revendication 1, dans lequel la concentration membranaire est une ultrafiltration.

8. Un procédé selon la revendication 1, dans lequel la protéine est une enzyme.

9. Un procédé selon la revendication 8, dans lequel l'enzyme est une oxydoréductase, une protéase, une lipase, une cellulase ou une amylase.
